# EUROPÄISCHE PATENTSCHRIFT

(11) **EP 1 263 422 B1**
(45) Veröffentlichungstag und Bekanntmachung des Hinweises auf die Patenterteilung: **08.03.2006**
(21) Anmeldenummer: 01925440.8
(22) Anmeldetag: 13.03.2001
(51) Int. Cl.: A61K 31/07, A61K 9/00, A61K 9/127

(54) **MITTEL ZUR BEHANDLUNG VON ERKRANKUNGEN DES TRACHEO-BRONCHIALTRAKTES, INSBESONDERE DER COPD**
AGENT FOR TREATING ILLNESSES OF THE TRACHEOBRONCHIAL TRACT, ESPECIALLY CHRONIC OBSTRUCTIVE PULMONARY DISEASE (COPD)
AGENT POUR LE TRAITEMENT DE MALADIES DU TRACTUS TRACHEOBRONCHIQUE, EN PARTICULIER DE LA BRONCHOPNEUMOPATHIE CHRONIQUE OBSTRUCTIVE

(30) Priorität: 13.03.2000 DE 10012151
(43) Veröffentlichungstag der Anmeldung: 11.12.2002
(73) Patentinhaber: GSF-Forschungszentrum für Umwelt und Gesundheit GmbH, 85764 Neuherberg (DE)
(72) Erfinder: FRANKENBERGER, Marion, 81479 München (DE); MAIER, Konrad, 81475 München (DE); SCHEUCH, Gerhard, 35285 Gemünden (DE); ZIEGLER-HEITBROCK, Hans-Werner, 82211 Herrsching (DE)
(74) Vertreter: Behnisch, Werner
(86) Internationale Anmeldenummer: PCT/EP2001/002832
(87) Internationale Veröffentlichungsnummer: WO 2001/068081

(56) Entgegenhaltungen:
- EP-A- 0 352 412
- EP-A- 0 450 991
- EP-A- 0 848 949
- WO-A-96/04891
- WO-A-98/25587

## Beschreibung

Die Erfindung betrifft ein Mittel zur Prophylaxe und Therapie von Erkrankungen des Tracheo-Bronchialtraktes, insbesondere der COPD (chronic obstructive pulmonary disease).

Die COPD tritt typischerweise bei Rauchern auf. Sie ist charakterisiert durch eine chronische Entzündung der kleinen Atemwege (< 2 mm), die unaufhaltsam zum Gewebsumbau und zur irreparablen Verengung (Obstruktion) dieses Teils der Atemwege führt.

Die feingewebliche Untersuchung dieser kleinen Atemwege zeigt eine vermehrte Schleimbildung, eine Zunahme der schleimbildenden Becherzellen, eine Hypertrophie der glatten Muskulatur, sowie eine Infiltration mit pigment-beladenen Makrophagen und mit CD8-positiven T-Lymphozyten. Mit Fortschreiten des Prozesses kommt es zum Gewebsumbau und zur verstärkten Ablagerung von Bindegewebsfasern.

Pathophysiologisch steht die Entzündung im Vordergrund. Hierbei infiltrieren neutrophile Granulozyten, Makrophagen und Lymphozyten das Gewebe der kleinen Atemwege. Diese Infiltration ins Gewebe wird durch die Bildung von Proteasen durch die Leukozyten erleichtert. Im Gewebe werden Mediatoren gebildet, die die Schleimbildung fördern, die Muskelzellen stimulieren und die Bildung von Bindegewebsfasern (Kollagen) durch Fibroblasten anregen.

In der atem-mechanischen Untersuchung zeigt die COPD einen verminderten maximalen exspiratorischen Fluss (FEV₁) und eine verlangsamte forcierte Entleerung der Lunge.
Die COPD ist oft mit einer chronischen Bronchitis und einem Emphysem vergesellschaftet. Diese beiden Erkrankungen sind jedoch klar von der COPD abgrenzbar.

Die chronische Bronchitis ist klinisch definiert durch einen chronisch produktiven Husten über mindestens 3 Monate pro Jahr in mindestens 2 aufeinander folgenden Jahren. Diese Erkrankung ist geprägt von meist bakteriellen Infektionen und sie kann, muß aber nicht zu einer irreversiblen Obstruktion führen.

Das Emphysem ist definiert als eine irreversible Erweiterung der Lufträume distal der terminalen Bronchien, bedingt durch einen Abbau der Alveolarsepten, verbunden mit einem Abbau von elastischen Fasern. Beim Emphysem tritt keine Fibrose auf.

Damit ist die COPD von der chronischen Bronchitis und dem Emphysem als Entität abgrenzbar, auch wenn die COPD mit diesen beiden Erkrankungen vergesellschaftet sein kann.
Die COPD betrifft vorwiegend die kleinen Atemwege, die chronische Bronchitis die großen und mittleren, das Emphysem die Alveolen.
Die COPD ist ein unaufhaltbarer Prozess der zum Gewebsumbau mit Fibrosierung (Faserzunahme) führt. Die chronische Bronchitis führt nicht zum Gewebsumbau. Beim Emphysem kommt es zur Destruktion der Alveolarwände, insbesondere der elastischen Anteile der extrazellulären Matrix.
Bei der COPD findet sich eine typische Zunahme von neutrophilen Granulozyten, Makrophagen und CD8-Zellen in den kleinen Atemwegen, bei der chronischen Bronchitis findet sich eine Beteiligung aller Entzündungszellen, beim Emphysem stehen die Leukozyten nicht im Vordergrund.

Aus der EP 0 352 412 ist bereits die Verwendung von Estern der Retinsäure und/oder einem Ester des Retinols in der Zubereitungsform eines Aerosol-Inhalats zur inhalativen topischen Anwendung für die Vorbeugung und Behandlung von Schleimhauterkrankungen des Tracheo-Bronchialtraktes von Mensch und Tier bekannt. Bei diesen sogenannten Bronchitiden handelt es sich um akute und chronische Bronchitiden, die keine Obstruktion zeigen. Beispielsweise leidet die Mehrzahl der Raucher an einer chronischen Bronchitis, zeigt jedoch keine Obstruktion der Atemwege und somit keine COPD. Bronchiektasen sind irreversible, zylindrische, sackförmige oder variköse Erweiterungen der Bronchien, ein Krankheitsbild, das nicht zur chronisch obstruktiven Erkrankung, der COPD, gehört.

Die in der EP 0 352 412 genannten Schleimhauterkrankungen des Tracheo-Bronchialtraktes sind somit keine unter den Begriff COPD subsumierbare Erkrankungen. Sie unterscheiden sich von der COPD insbesondere dadurch, dass die COPD-typische irreversible Obstruktion fehlt.

Die COPD wurde bisher beispielsweise durch Gabe von ß-Adrenergika/Anticholinergika, Theophyllin, und/oder Glukocortikoiden behandelt. Nachteile der genannten Medikamente sind, dass sie nur symptomatisch wirken und keinen Einfluss auf den schicksalhaften Verlauf der COPD haben. Spasmolytika wirken lebensverkürzend. Theophyllin verursacht Herzrythmusstörungen. Eine wichtige Nebenwirkung von Glukocortikoiden ist die Osteoporose.

Die EP 0 450 991 beschreibt eine Phospholipide enthaltende Zusammensetzung zur Behandlung von obstruktiven Erkrankungen der Atemwege, entweder alleine oder in Verbindung mit weiteren Exzipienten. Als weitere Wirkstoffe werden unter anderem Retinsäurederivate genannt. Als Phospholipide werden Phosphatidylcholine, Phosphatidylglycerine und Phosphatidylsäuren genannt. Die Phospholipide liegen in Form mizellarer Suspensionen oder liposomaler Suspensionen, auch als Aerosole, vor.

Die WO 98/25587 beschreibt die Verwendung lipophiler antioxidativer Verbindungen zur Verhinderung von durch Retinsäure induzierten, UVA-vermittetten oxidativen Reaktionen, wobei ein Retinoid und eine wirksame Menge eines lipophilen Antioxidationsmittels typisch auf die Haut aufgebracht werden.

Die WO 96/04891 beschreibt die Verwendung einer Carotinoide enthaltenden Zusammensetzung, die in Lipidträgerteilchen vorliegt, zur Behandlung von Tumorerkrankungen. Als Carotinoid wird beispielsweise die Retinsäure genannt.

Die EP 0 848 949 beschreibt eine pharmazeutische Zusammensetzung in Form eines Aerosols zur Anwendung auf Schleimhäuten der Atemwege, enthaltend einen Wirkstoff aus der Gruppe der Retinoide und Carotinoide, ein verflüssigtes Treibgas oder Treibgasgemisch sowie ggf. antioxidativ wirkende und/weiter übliche pharmazeutische Hilfsstoffe.

Es ist eine Aufgabe der vorliegenden Erfindung, ein neues Mittel zur Behandlung der COPD bereitzustellen, welches die aus dem Stand der Technik bekannten Nachteile vermeidet.

Die Aufgabe wird erfindungsgemäß dadurch gelöst, daß Vitamin A und/oder seine Derivate und/oder Ester in einer therapeutisch wirksamen Menge zur Therapie der COPD eingesetzt werden, die in Form eines Aerosol-Inhalats appliziert werden. Die weiteren Merkmale des Zusammensetzung ergeben sich aus Anspruch 1.

Unter den Ausdruck "Derivate" des Vitamin A fallen Retinol, Retinal und Retinsäure, je mit ihren Derivaten und Estern, sowie β-Carotin und seine Derivate als Vorstufe von Vitamin A. Beispielsweise kann Retinol mit Linolsäure, Stearinsäure, Palmitinsäure, Ölsäure als gesättigte und ungesättigte Fettsäuren verestert sein. Dies schließt auch chemische Modifikationen der genannten Fettsäuren und Alkohole mit ein. (siehe auch EP 0 352 412 B1; S.11 Anspruch 4 + Anspruch 6). Beispielhaft sei verwiesen auf Martindale, The Extra Pharmacopeia, 1982, London, The Pharmaceutical Press, und alle Folgeauflagen. Auf dieses Dokument wird vollinhaltlich verwiesen.

Zur inhalativen Verabreichung wird das Vitamin A in Aerosolform appliziert, wozu insbesondere Sprays und Inhalate einsetzbar sind. Hierdurch wird der Wirkstoff zu kleinsten Partikeln zerteilt und an den Ort seiner therapeutischen Wirksamkeit transportiert.

Das Vitamin A und seine Derivate und Ester liegen erfindungsgemäß in Aerosolform vor. Aerosole sind feine Nebel, die im allgemeinen durch unter Druck stehende Systeme erhalten werden. Sie können beispielsweise durch Versprühen oder Vernebeln von Flüssigkeiten, aber auch von Trockenpulvern, erhalten werden. Inhalationsaerosole unterscheiden sich von üblicherweise als Aerosole bezeichneten Systemen dadurch, daß die Aerosol-Partikel in einer Größenordnung von unter 10 µm, bevorzugt im Bereich von 1-10 µm, vorliegen.

Die grundlegenden Bestandteile von Aerosol-Systemen in Sprayform sind beispielsweise ein Behälter mit einem Treibmittel, wobei zwischenzeitlich auch treibmittellose Aerosolsysteme entwickelt wurden. Weiterhin enthalten sind im Aerosolsystem der oder die Wirkstoffe zusammen mit üblichen Bgleitstoffen wie beispielsweise Treibmitteln und Laktose. Die Zusammensetzung dieser Bestandteile bestimmt die Eigenschaften des Aerosol-Inhalats, beispielsweise also die Teilchengrößenverteilung, die Zufuhrrate, die Viskosität usw.

Aerosole können als Zweiphasenaerosole (Gas und Flüssigkeit) oder Dreiphasenaerosole (Gas, Flüssigkeit und Feststoff oder Flüssigkeit) vorliegen. Zweiphasenaerosole bestehen aus einer Lösung der Wirkstoffe in einem verflüssigten Treibgas und dem vernebelten Treibgas. Das Lösungsmittel besteht beispielsweise aus dem Treibgas oder einer Mischung aus dem Treibgas und Co-Lösungsmitteln wie Alkohol, Propylenglycol und Polyethylenglycolen, die häufig zur Verbesserung der Löslichkeit der Wirkstoffe eingesetzt werden.

Dreiphasensysteme bestehen aus einer Suspension oder Emulsion des Wirkstoffs bzw. der Wirkstoffe zusammen mit den vernebelten Treibgasen. Eine Suspension besteht aus dem Wirkstoff, dispergiert in dem Treibgassystem mit Hilfe üblicher Hilfsstoffe, beispielsweise Benetzungsmitteln und/oder festen Trägerstoffen wie Talkum oder kolloidaler Kieselsäure.Treibgase sind bekannt und umfassen verschiedene Kohlenwasserstoffe, möglichst ohne die Ozonschicht schädigende Wirkungen.

Eine Inhalation ist nur möglich, wenn die Inhalationslösungen zu feinsten Partikeln im Größenordnungsbereich von 1 - 10 µm zerstäubt werden. Nur derartige feinverteilte Aerosole sind überhaupt inhalierbar. Bei üblichen Aerosolen ist eine Inhalation kontraindiziert. Weitere Informationen über Aerosol-Inhalate sind beispielsweise in der US Pharmacopeia aufgeführt, auf die hier vollinhaltlich Bezug genommen wird.

In einer bevorzugten Ausführungsform der Erfindung liegt das Vitamin A in Liposomen eingeschlossen vor. Liposomen sind wäßrige Kompartimente, die von einer völlig geschlossenen Lipid-Doppelschicht umgeben sind. Liposomen werden beispielsweise durch Suspendieren geeigneter Lipide in wäßrigen Lösungen künstlich erzeugt. Die Liposomen werden dispergiert, so daß ungefähr gleich große geschlossene Vesikel entstehen. In die wäßrigen Lösungen bzw. die Lipidmembranen können dann Fremdmoleküle, erfindungsgemäß Vitamin A, eingebaut werden. Es hat sich gezeigt, daß liposomal verpacktes Vitamin A den therapeutischen Effekt steigern und die Nebenwirkungen effizient verringern kann. Einzelheiten hierzu werden nachfolgend näher beschrieben.

Es konnte gezeigt werden, daß Liposomen, die zumindest Phosphatidylcholin mit zumindest einem weiteren Phospholipid, ausgewählt aus Phosphatidylinositol, Phosphatidylserin, Phosphatidinsäure und Cardiolipin, enthalten, sich zur Behandlung der COPD besonders eignen. Die Verwendung dieser speziell zusammengesetzten Liposomen bewirkt eine selektive Aufnahme durch Alveolarmakrophagen, eine höhere Spezifität bei der Therapie und eine Verringerung der therapeutisch wirksamen Dosis verbunden mit einer Reduzierung der Nebenwirkungen.

Der Anteil von Phosphatidylcholin zum Anteil der übrigen Phospholipide beträgt 65: 35-75: 25 je bezogen auf 100% Phospholipide. Phosphatidylcholin ist somit ein essentieller Bestandteil der Liposomen, wobei der Rest, ein oder mehrere der obengenannten Phospholipide sein können. Die Liposomen der vorliegenden Erfindung enthalten weiterhin Antioxidantien in einem Anteil von 10 - 50%. Bevorzugt wird hier Vitamin E oder deren Derivate, wie zum Beispiel (+)-α Tocopherol Acetat, (±)-α Tocopherol Acetat, (±)-α Tocopherol Acid Succinate eingesetzt. Der Anteil der Phospholipide beträgt 50% - 90%.

Die Menge an Vitamin A, die in die Liposomen eingeschlossen wird, kann vom Fachmann durch übliche medizinisch-therapeutische Reinversuche, die selbstverständlich im Vorfeld an Tieren durchgeführt werden müssen, bestimmt werden. Auch die beschriebene LiposomenFormulierung wird sinnvollerweise als Aerosol-Inhalt eingesetzt, um die optimale therapeutische Wirkung zu erzielen.

Vitamin A-Zubereitungen als Aerosol-Inhalat in der hier beschriebenen, speziell zusammengesetzten Liposomenform, wurden bisher nicht beschrieben. Eine derartige pharmazeutische Zubereitung kann nicht nur zur Behandlung der COPD, sondern auch beispielsweise zur Behandlung chronischer Bronchitiden, von Emphysemen und malignen sowie benignen Lungenerkrankungen eingesetzt werden allgemein zur Behandlung von Erkrankungen des Tracheo-Bronchialtraktes.

Die erfindungsgemäß eingesetzten Liposomen unterscheiden sich neben ihrer Zusammensetzung noch dadurch von den aus dem Stand der Technik bekannten multilamellären Liposomen, daß sie unilamellär vorliegen. Die spezielle Kombination aus Liposomenaufbau, Wirkstoff und Struktur führt zu der erfindungsgemäß gefundenen überraschenden Wirksamkeit bei der Behandlung von Erkrankungen des Tracheo-Bronchialtraktes.

Bevorzugt werden solche Phospholipide eingesetzt, die ein selektives Targeting von Alveolarmakrophagen durch Bindung an spezifische, auf Alveolarmakrophagen vorhandene Rezeptoren, den sogenannten Scavenger-Rezeptoren, ermöglichen. Hierzu gehören die nachfolgenden Phospholipide:

### 1. Phosphatidylinositol (1,2-Diacyl-sn-glycero-3-phospho-[1-D-myo-inositol])

Die Acylreste können sich von der Arachidonsäure oder von der Linolsäure, Palmitinsäure, Stearinsäure und anderen ungesättigten bzw. gesättigten Fettsäuren ableiten

### 2. Phosphatidylserin (1,2-Diacyl-sn-glycero-3-phospho-serin)

Die Acylreste können sich von der Arachidonsäure oder von der Oleinsäure, Palmitinsäure und anderen ungesättigten und gesättigten Fettsäuren ableiten.

### 3. Phosphatidinsäure (1,2-Diacyl-sn-glycero-3-phosphate)

Die Acylreste können sich von der Arachidonsäure oder von der Stearinsäure, Decanoylsäure, Heptadecanoylsäure, Laurylsäure, Myristoylsäure, Octanoylsäure, Oleylsäure, Palmitinsäure und anderen ungesättigten und gesättigten Fettsäuren ableiten

### 4. Cardiolipin (Diphosphatidylglycerol)

Cardiolipin enthält maximal 4 Acyl-Reste, die sich von ungesättigten und gesättigten Fettsäuren ableiten.

### 5. Phosiphatidylcholin (L-α-Lecithin, 1,2-Diacyl-sn-glycero-3-phosphocholine) (partiell oxidiert)

Die Acylreste können sich von der Arachidonsäure oder von ungesättigten und gesättigten Fettsäuren ableiten. Mindestens 1 Acyl-Ligand ist dabei ungesättigt. Der ungesättigte Acyl-Rest ist partiell oxidiert und vermittelt die Bindung der Liposomen an Scavenger-Rezeptoren.

Die erfindungsgemäß eingesetzten Liposomen weisen eine Größe im Bereich von 0.1 µm bis 2 µm, insbesondere bevorzugt von 0,4 µm auf. Es werden unilamelläre Liposomen eingesetzt. Die Herstellung erfolgt durch eine Art Hochdruckfiltration, beispielsweise mit einem kommerziell erhältlichen Extruder der Firma Lipex Biomembranes, Kanada. Herkömmliche Liposomen, wie sie beispielsweise in dem Artikel von Parthasarathy et al, Cancer Chemother. and Pharmacol. 1999, 43(4), 277-283, beschrieben sind, werden durch Rekonstitution eines trockenen Lipidfilms in der gewünschten Flüssigkeit hergestellt. Hierbei entstehen sogenannte multilamelläre Liposomen, d.h. Liposomen mit mehreren Lipidschichten um den wäßrigen Kern. Derartige Liposomen sind in ihrer Größe recht heterogen und können, wie in Parthasarathy et al gezeigt, durch Ultraschall auf eine einheitliche Größe gebracht werden. Sie bleiben hierdurch aber weiterhin multilamellär. Die in Parthasarathy et al beschriebenen multilamellären Liposomen neigen jedoch zu einer Aggregatbildung oder verschmelzen zu noch größeren Liposomen. Die Verwendung derartiger Liposomen hat sich erfindungsgemäß als ungeeignet herausgestellt. Durch die Verwendung des oben beschriebenen Extruders, der nur beispielsweise genannt ist, können zum einen die fertig rekonstituierten Liposomen vor ihrer Verwendung extensiv gewaschen werden, d.h. nicht eingebaute Substanzen werden entfernt, zum anderen werden die unilamellären Liposomen, die nur eine Lipidschicht um den wäßrigen Kern aufweisen, weiterverarbeitet, was ihnen eine besondere Stabilität verleiht. Gerade die Kombination aus der hier beschriebenen Lipidzusammensetzung und der Verwendung unilamellärer Liposomen führt zu einer besonders bemerkenswerten therapeutischen Effizienz.

Nur die erfindungsgemäß bereitgestellte Liposomenzubereitung ermöglicht die Lösung der Aufgabe, nämlich den Wirkstoff an die spezifischen Zielzellen, nämlich die Alveolarmakrophagen, heran zu führen. Nur diese spezielle Zusammensetzung der Liposomen gewährleistet, daß sie von bestimmten Zielzellen erkannt und von diesen aktiv aufgenommen wird. Auf diese Weise ist es möglich, eine bisher nicht bekannte und nicht beschriebene Wirkung von Vitamin A in Alveolarmakrophagen als relevante Zielzellen zu erzeugen.

Durch die erfindungsgemäße pharmazeutische Zusammensetzung erfolgt ein sehr spezifischer Eingriff in den Stoffwechsel einer bestimmten Art von Leukozyten in der Lunge, nämlich den Alveolarmakrophagen, um den Entzündungsprozeß, insbesondere der COPD, nachhaltig zu beeinflussen.

Im Stand der Technik war unbekannt, daß ein zelluläres Targeting durch die besondere liposomale Zusammensetzung möglich ist, um neue, therapeutische Möglichkeiten zu öffnen. Das zelluläre Targeting der erfindungsgemäß gewählten Liposornenzusammensetzung ist Voraussetzung zur Lösung der erfindungsgemäß gestellten Aufgabe in anderen, d. h. nicht Alveolarmakrophagen, ist eine andere, für das oben genannte Ziel sogar abträgliche Wirkung durch Vitamin A denkbar. Eine solche Wirkung ist beispielsweise an Osteoblasten der Ratte beschrieben worden.

In Kirilenko V.N. und Gregoriadis G., J. Drug Targeting 1993, 1 (4), 361 - 368 werden Lipide als Emulgatoren beschrieben, die die unspezifische Resorption von Vitaminen im Rahmen der Nahrungsaufnahme verbessern. Die besondere, erfindungsgemäß eingesetzte Zusammensetzung wird nicht beschrieben und die Verwendung zur Behandlung von Erkrankungen des Tracheo-Bronchialtraktes insbesondere der COPD, nicht nahegelegt. Nur die spezielle, hier eingesetzte Zusammensetzung enthält die Möglichkeit, daß Vitamin A an bestimmte Zielzellen transportiert wird und von diesen aktiv aufgenommen werden kann.

In der EP-A-0 229 561 werden Liposomen als Träger eingesetzt, um Vitamin A an Hautzellen heran zu bringen. Die verwendete Zusammensetzung weist ausschließlich kosmetische, aber keine therapeutische Wirkungen auf. Die Verwendung im Tracheo-Bronchialtrakt dieser Zusammensetzungen ist undenkbar. Die in der EP-A-0 229 561 genannten Liposomen besitzen auch nicht die Eigenschaft, eine besondere Zielzelle in der Lunge zu erkennen, um von dieser Zelle hochspezifisch aufgenommen zu werden.

Auch die EP-A-0 352 412 nimmt den Erfindungsgegenstand nicht vorweg und legt ihn auch nicht nahe. Zwar wird hier die Behandlung des Bronchialepithels durch Vitamin A beschrieben, jedoch nicht unter Verwendung der speziellen liposomalen Zubereitung der Erfindung.

Nachfolgend wird die Erfindung anhand der beiliegenden Abbildungen näher beschrieben. Die Abbildungen zeigen:

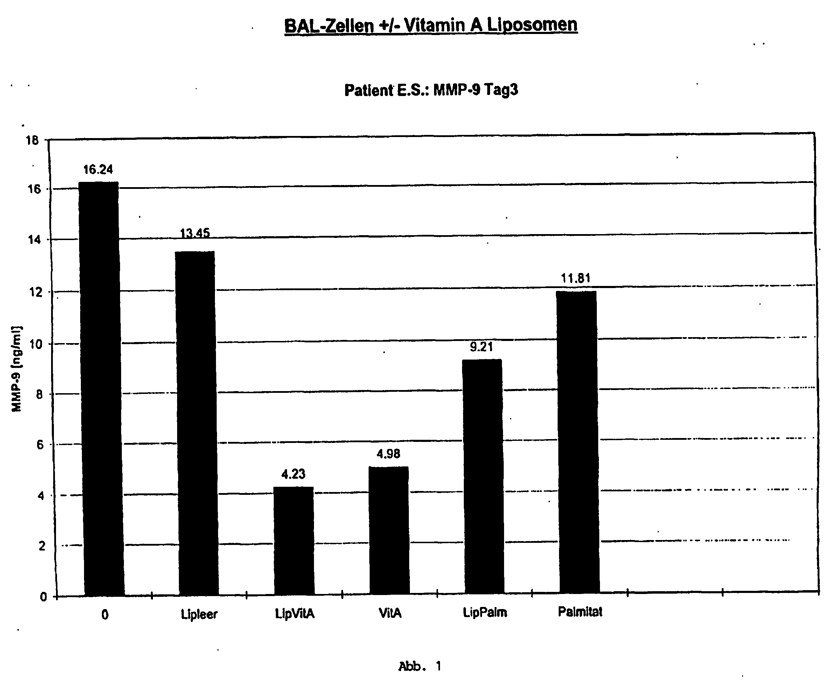

- Abbildung 1:: BAL-Zellen +/- Vitamin A Liposomen - Patient E.S.: MMP-9 Tag 3;

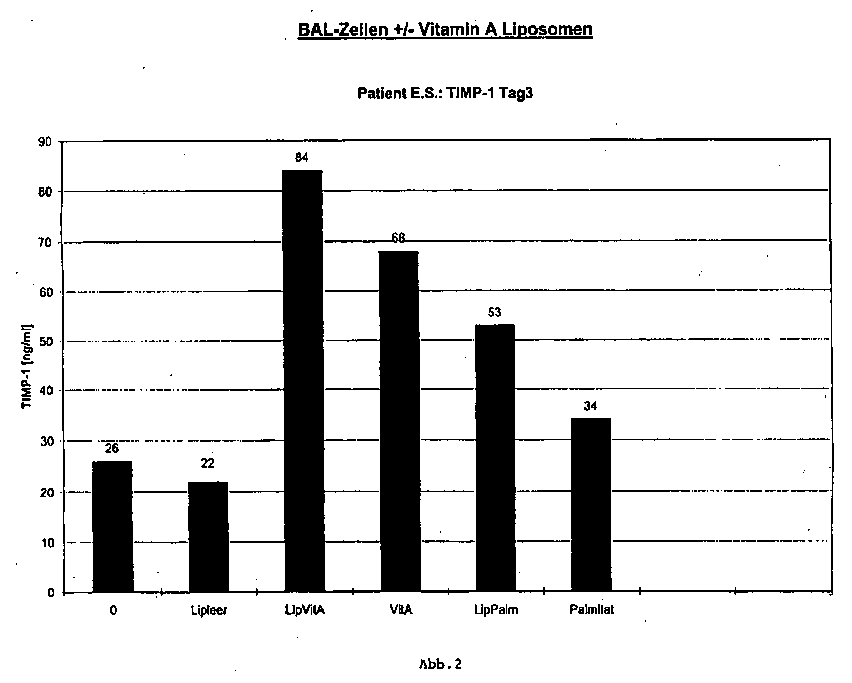

- Abbildung 2:: BAL-Zellen +/- Vitamin A Liposomen - Patient E.S.: TIMP-1 Tag 3;

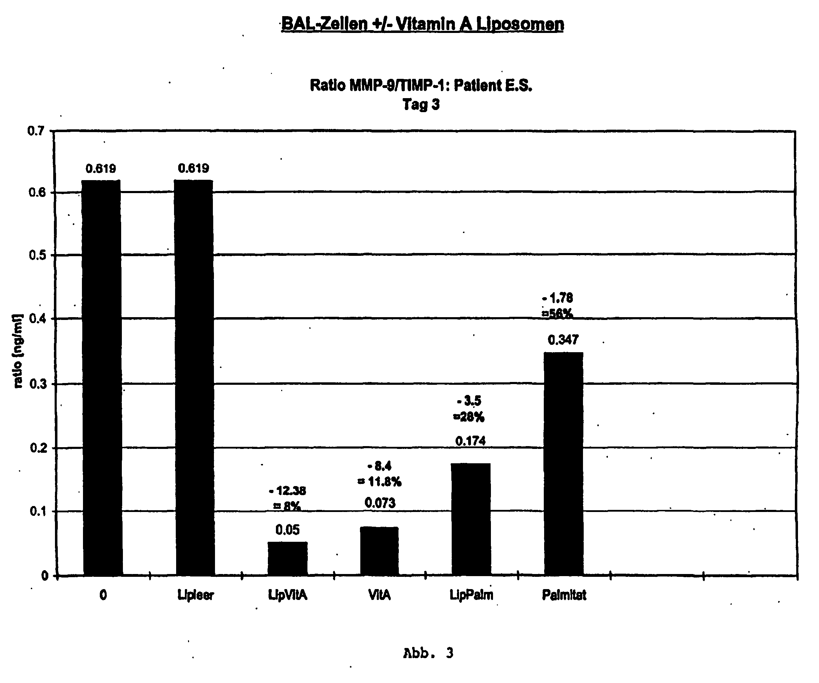

- Abbildung 3:: BAL-Zellen +/- Vitamin A Liposomen Ratio MMP-9/TIMP-1: Patient E.S.Tag 3;

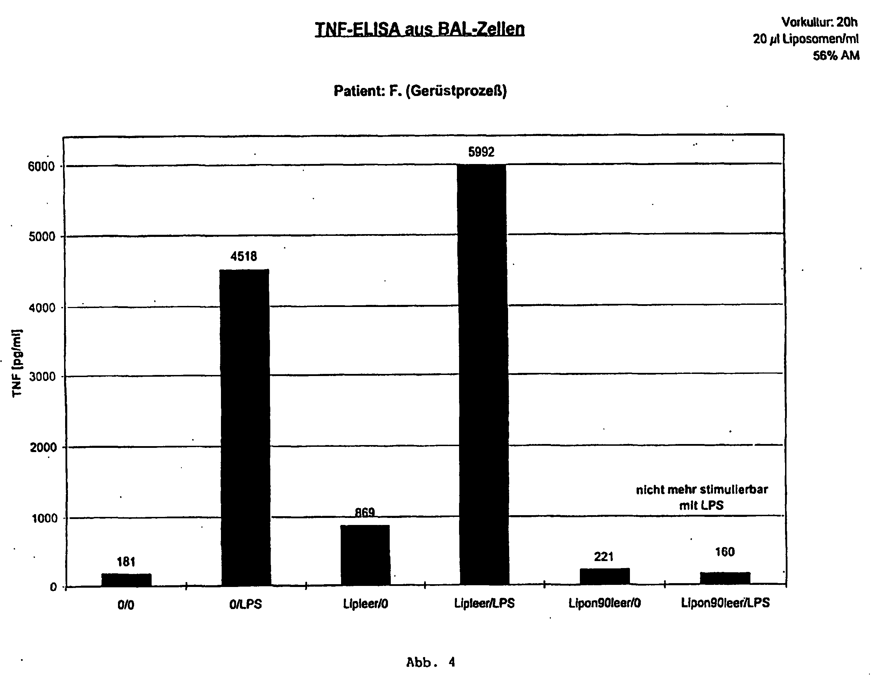

- Abbildung 4:: TNF-ELISA aus BAL-Zellen Patient: F. (Gerüstprozeß)

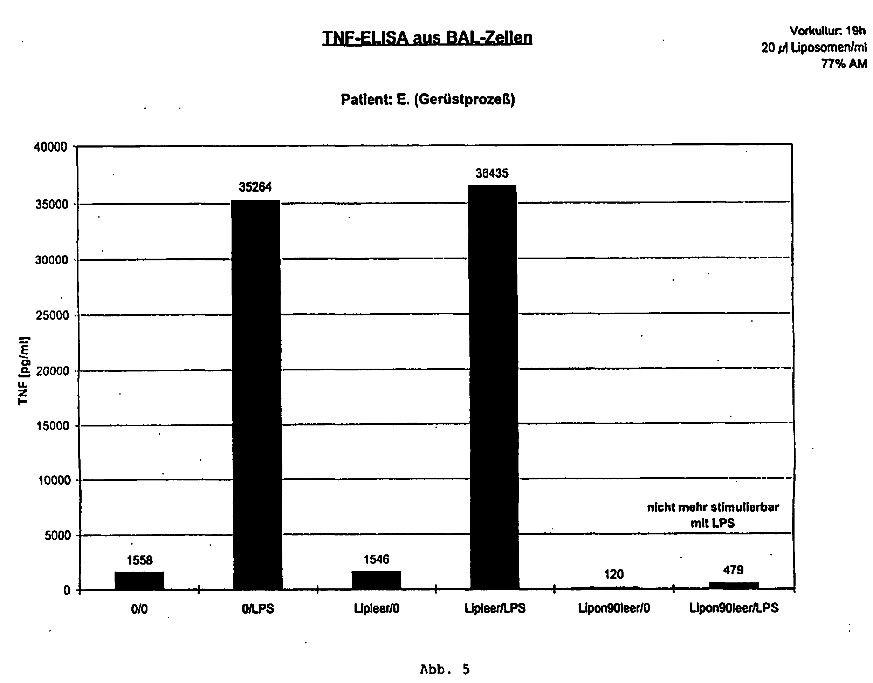

- Abbildung 5:: TNF-ELISA aus BAL-Zellen Patient: E. (Gerüstprozeß)

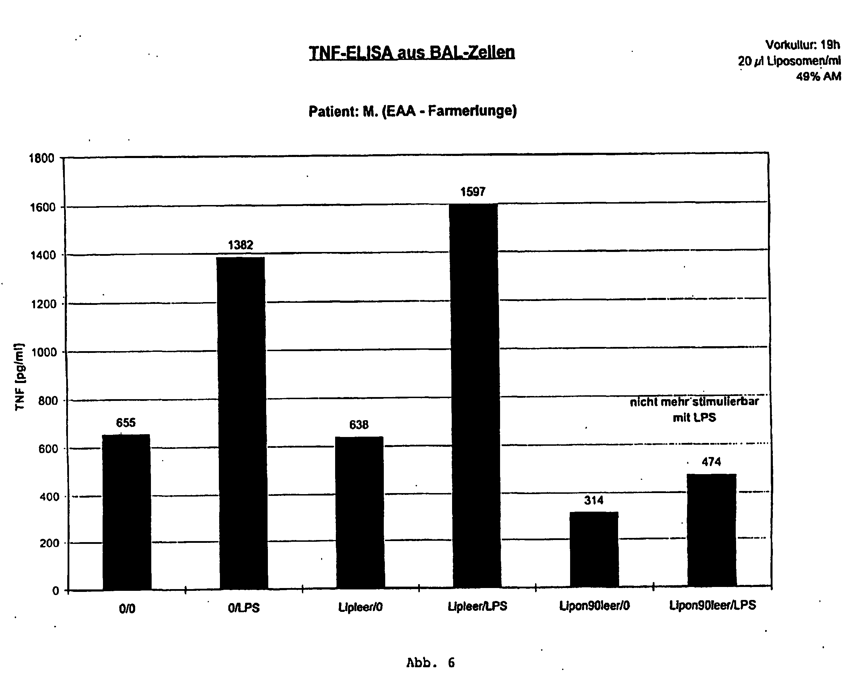

- Abbildung 6:: TNF-ELISA aus BAL-Zellen Patient: M. (EAA - Farmerlunge)

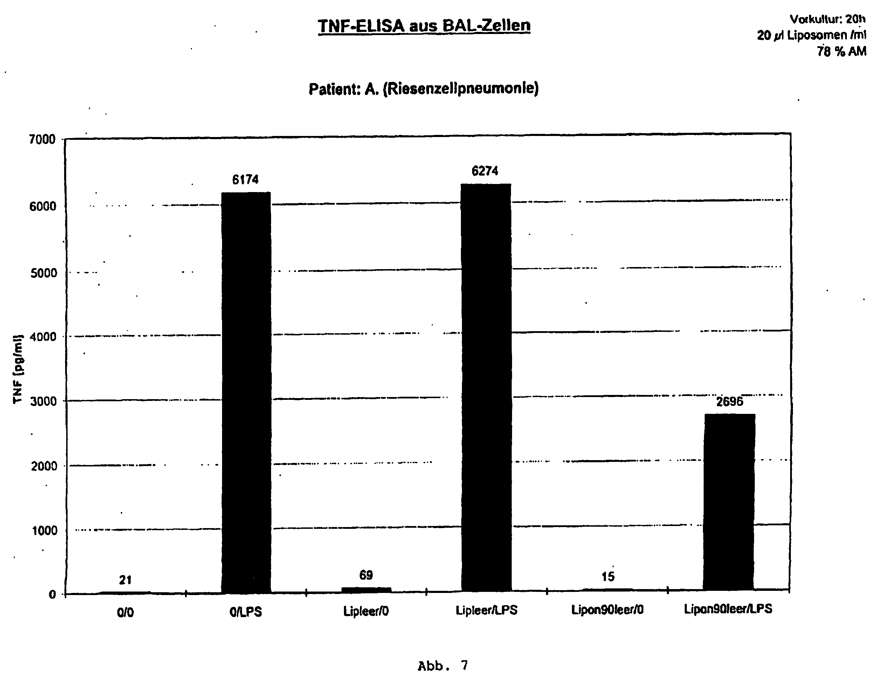

- Abbildung 7:: TNF-ELISA aus BAL-Zellen Patient: A. (Riesenzellpneumonie)

- BAL: = Bronchioalveoläre Lavage
- TNF: = Tumornekrosefaktor
- AM: = Alveolarmakrophagen
- ELISA: = Enzyme Linked Immunoabsorbent Assay

Nachfolgend wird die Erfindung anhand von Ausführungsbeispielen näher beschrieben. Die Erfindung ist jedoch nicht auf die nachfolgenden Beispiele beschränkt.

Es gibt zunehmend Hinweise dafür, daß Störungen im Proteasen/Proteasen-Inhibitoren-Gleichgewicht ursächlich an der Pathogenese chronisch verlaufender Lungenerkrankungen beteiligt sind. So konnte gezeigt werden, daß die von Alveolarmakrophagen sezemierten Matrix-Metalloproteasen (MMPs) irreversible Schädigungen am Lungengewebe verursachen und damit respiratorische Funktionseinschränkungen bewirken. Davon sind insbesondere Patienten mit chronisch obstruktiver Bronchitis (COPD) betroffen. Bei COPD wird von den MMPs insbesondere die Metalloprotease MMP-9 vermehrt in den Atemwegen und den Alveolen sezerniert, die durch den natürlichen Antagonisten TIMP-1 (tissue inhibitor of metalloprotease) nicht vollständig neutralisiert werden kann. Als Hauptproduzent der MMP-9 in der Lunge gilt der Alveolarmakrophage, der in neuesten Untersuchungen an der Ratte als Hauptverantwortlicher bei der Emphysembildung diskutiert wird (nicht wie bislang angenommen die neutrophilen Granulozyten). Eigene Untersuchungen zeigen eine Erhöhung der MMP-9 auch im Serum von COPD-Patienten. Entscheidendes Kriterium für das Ausmaß einer Störung der Proteasen-Antiproteasen-Balance ist das Verhältnis zwischen Proteinase und Inhibitor: MMP-9/TIMP-1.

Erfindungsgemäß konnte in einem in-vitro-System mit Alveolarmakrophagen gezeigt werden, daß Vitamin A nicht nur die Expression von MMP-9 signifikant hemmt, sondern, was ebenfalls völlig überraschend war, gleichzeitig die Expression des spezifischen Inhibitors TIMP-1 signifikant stimuliert. Diese Effekte waren am deutlichsten erkennbar, wenn Vitamin A zuvor in die oben beschriebene, speziell zusammengesetzten Liposomen verpackt wurde, was eine spezifische Aufnahme durch die Alveolarmakrophagen sicherstellte.

Durch die Verwendung von liposomal verpacktem Vitamin A konnte das Verhältnis MMP-9/TIMP-1 unerwartet deutlich, nämlich ca. um den Faktor 12, reduziert werden. Dies bedeutet eine effiziente Verringerung des gewebsschädigenden Potentials des proteolytischen Systems durch Vitamin A. Auf der Grundlage dieser Untersuchungen wurde der vorliegende Therapieansatz zur Behandlung der COPD und des Lungenemphysems etabliert. Die Zusammensetzung der Liposomen wurde vorstehend bereits beschrieben. Die genaue Herstellung der Liposomen wird in den nachfolgenden Ausführungsbeispielen wiedergegeben.

Die Wirkung von Vitamin A auf Metalloproteasen ist bekannt. Jedoch sind die Ergebnisse, die durch die Verabreichung von Vitamin A erzielt werden, äußerst gegensätzlich. Während in einigen Untersuchungssystemen eine Verringerung der Metalloproteasenkonzentration durch eine Hemmung der Expression der für sie kodierenden Gene beschrieben wird, wird in anderen Arbeiten eine erhöhte Expression und damit eine erhöhte Konzentration von Metalloproteasen beschrieben. In Park and Kim, Mol. Cells 1999, 9(2):119-26 wird beschrieben, daß durch den Einfluß der Retinsäure Enzyme mit Gelatinase-Aktivitäten, die zu den Metalloproteasen zu rechnen sind, in ihrer Aktivität erhöht waren, insbesondere die Expression dieser Metalloproteasen erhöht wurde. In der Veröffentlichung von Overall, Journal of Cellular Physiology, 1995, 164(1):17-25 wird beschrieben, daß Retinsäure die Expression von Metalloproteasen in Osteoblasten der Ratte erhöht, während gleichzeitig die mRNA-Spiegel von TIMP-1 in Abhängigkeit von der verabreichten Retinsäuredosis deutlich reduziert wurden. In der Arbeit von Heath et al., Biochemical and Biophysical Research Communications 1990, 168(3):1171-1176, wird beschrieben, daß unter dem Einfluß von Retinol die TIMP-Spiegel auf 0 fielen.

Unter Berücksichtigung des oben beschriebenen Standes der Technik ist es für den Fachmann ein überraschendes Ergebnis, daß unter dem Einfluß von Vitamin A, also der Retinsäure und des Retinols, nicht nur eine deutliche Hemmung der Expression der Metalloprotease MMP-9 in Alveolarmakrophagen und Blutmonozyten erreicht wird, sondern gleichzeitig auch eine Stimulation des TIMP-1-Inhibitors.

Im Rahmen der Experimente zur Aufnahme der erfindungsgemäßen Liposomen durch Monozyten im Blut wurden als Testsysteme auch die verschiedensten gängigen Zellinien unterschiedlichen Ursprungs bezüglich ihrer Fähigkeit zur Liposomenaufnahme überprüft. Dabei kamen vor allem Zellen myelomonozytären Ursprungs zur Anwendung, wie zum Beispiel die Zellinien HL-60, U937, THP-1 und Mono Mac 6 (in ihrer Aufzählung nach aufsteigendem Reifegrad sortiert). So ist die monozytäre humane Zellinie Mono Mac 6 in ihrem Reifegrad am weitesten in Richtung Makrophage entwickelt. Aber gerade diese Zelle hat kaum Liposomen aufgenommen, was gegen die Vorstellung spricht, daß eine weiter ausdifferenzierte Zelle auch eine höhere Expression von Phosphatidylserin-Rezeptor aufweist. Insofern war auch nicht naheliegend, daß Alveolarmakrophagen selektiv diese Liposomen aufnehmen werden. Zudem war nicht vorherzusagen, zu welchem Typ Makrophage sich ein Blutmonozyt entwickeln wird, denn es gibt erhebliche Unterschiede nicht nur bezüglich Morphologie und Funktion. Weiterhin war nicht bekannt, welche Zellen mit welcher Art von Phosphatidylserinrezeptor ausgestattet sind. Insbesondere war unbekannt, daß auf Alveolarmakrophagen ein Phosphatidylserinrezeptor vorliegt. Demnach war nicht nur die Wirkung von Vitamin A bei der Behandlung der COPD und des Lungenemphysems überraschend, sondern auch die erhöhte Wirksamkeit von Vitamin A durch die Verabreichung in Form von Liposomen, insbesondere durch speziell zusammengesetzte Liposomen, wie sie vorliegend beschrieben wurden.

Durch die Behandlung der COPD und des Lungenemphysems mit Vitamin A kann das Ungleichgewicht zwischen MMPs und TIMPs behandelt werden, d.h. die Expression ersterer wird gehemmt und die Expression letzterer stimuliert.

Die hier beschriebene Therapie wirkt der Gewebedestruktion entgegen und bietet einen neuen vielversprechenden Ansatz zur Behandlung chronisch obstruktiver Lungenerkrankungen. Die besonderen Vorteile der Erfindung liegen u.a. in:
- Verwendung einer geringeren Dosis, da eine selektive Aufnahme des liposomal verpackten Vitamin A durch die Alveolarmakrophagen, die die wichtigste Quelle der Metalloproteasen darstellen, erfolgt.
- Durch die gleichzeitige Beeinflußung von zwei Agonisten im Gleichgewicht des proteolytischen Systems der Lunge wird eine hocheffiziente Wirkung von Vitamin A erzielt.
- Das Proteasen-Antiproteasen-Gleichgewicht in der Lunge wird normalisiert.
- Das liposomal verpackte Vitamin A wird in Form eines Depots gespeichert;
- hierdurch wird eine geringere Menge von Vitamin A in den Blutkreislauf abgegeben, die Wirksubstanz bleibt direkt am Wirkort, der Lunge, und die systemischen Nebenwirkungen werden verringert.

Die nachfolgenden Versuche belegen die überraschenden Wirkungen der Verabreichung von Vitamin A, insbesondere in liposomaler Form. Hierzu wurden humane Alveolarmakrophagen (AM) über eine bronchoalveoläre Lavage gewonnen. Die AM wurden in Mono Mac 6 Medium + 10% FCS zu je 1 x 10⁶ Zellen in 1 ml Volumen pro Ansatz auf einer 24 well "low attachment " Platte (Costar) ausgesät. Es erfolgte die Zugabe der Vitamin A Liposomen bzw. der puren Substanzen. Es wurden aus parallelen Ansätzen jeweils am Tag 1, Tag 3 und Tag 4 Zellen für die RT-PCR bzw. der Kulturüberstand für die Bestimmung von Protein im ELISA geerntet. Im einzelnen wurden folgende Ansätze durchgeführt:
- β = Kontrollansatz, nur AM in Medium
- Lipleer = Liposomen leer
- LipVitA = Liposomen mit Vitamin A (Retinsäure, Sigma R-2625) 5 µM final
- VitA = Vitamin A pur (Retinsäure, Sigma R-2625) 5 µM final
- LipPalm = Liposomen mit all trans-Retinolpalmitat (Sigma R-3375) 3 µM final
- Palmitat = all trans-Retinolpalmitat pur (Sigma R-3375) 3 µM final

Die Kulturüberstände wurden mit handelsüblichen ELISA-Kits (Amersham Pharmacia) auf den Gehalt an MMP-9 und TIMP-1 hin überprüft. Die Angaben verstehen sich jeweils in ng/ml Kulturüberstand bezogen auf 1 x 10⁶ Zellen.

Die angegebenen Daten zeigen die Ergebnisse an Tag 3 nach Vitamin A Zugabe. An Tag 1 war auf Proteinebene noch kein Effekt erkennbar (wohl aber auf mRNA-Ebene); die Daten von Tag 4 entsprechen denen am Tag 3: während durch liposomales und freies Vitamin A die MMP-9 Expression stark reduziert wird, ist gleichzeitig ein Anstieg an TIMP-1 zu verzeichnen. Der Effekt des liposomalen Vitamin A ist dabei dem freien stets überlegen. Diese gegenläufige Regulation von MMP-9 und TIMP-1 erklärt auch den enormen Effekt auf die Ratio MMP-9/TIMP-1 durch Vitamin A Behandlung.

Die Kultur der BAL-Zellen und der PBMC mit liposomalem Vitamin A erfolgte wie folgt:

### Material:

- RPMI 1640 (Biochrom # F1415, Berlin), L-Glutamine 2mM (Gibco # 25030-024), Penicillin 200 U/ml Streptomycin 200 µg/ml (Gibco # 15140-114), non-essential amino acids (NEAA) 1-2 x (Gibco # 11140-35), OPI-Supplement (enthält Oxalacetat, Na-Pyruvat und Insulin) 10 ml für 11tr (Sigma # O-5003). Nach Filtration durch einen Gambro Ultrafilter U 2000 (Martinsried, Germany), um LPS zu entfernen, Zugabe von 10% FKS, LPS getestet.
- Costar low attachment 24 well Platten # 3473 (Bodenheim, Germany)

Pro Ansatz werden 1 x 10⁶ BAL-Zellen (= Zellen aus bronchoalveolärer Lavage, mit ca. 80% Alveolarmakrophagenanteil) bzw. 1.5 x 10⁶ PBMC (= peripheral blood mononuclear cells, isoliert über LymphoPrep-Gradient (Nycomed, Norwegen)) auf 1 ml Volumen verwendet. Die 24 well low attachment Zellkulturplatte nach den Angaben des Herstellers vorbehandeln. Dazu die benötigten Vertiefungen mit 1 ml Medium füllen und 20 min bei 37 °C im Brutschrank inkubieren. Danach das Medium wieder abnehmen und durch die Zellsuspension ersetzen. Die mit Vitamin A beladenen Liposomen in einer Endkonzentration von 5 x 10⁻⁶ M zugeben. Zur Kontrolle Parallelansätze mit purem Vitamin A und leeren Liposomen durchführen. Die Zellen für die Messung von MMP-9 und TIMP-1 im Kulturüberstand und die Bestimmung der mRNA Expression für drei Tage bei 37 °C im Brutschrank inkubieren. Die Zellen danach gut aufsuspendieren und zunächst pro PCR-Lysat 2 × 10⁴ Zellen entnehmen und in einem Eppendorf Reaktionsgefäß in 200 µl RNAclean (AGS, Heidelberg, Germany) lysieren. Diese PCR-Lysate bis zur Verwendung bei -20 °C lagern. Die restliche Zellsuspension in ein Eppendorf-Gefäß überführen und 5 min bei 14.000 x g abzentrifugieren. Den Überstand abnehmen, in ein frisches Gefäß geben und bis zur Messung bei -80 °C lagern.

### Ansätze im Überblick:

| | |
|---|---|
| 1. BAL-Zellen bzw. PBMC | ohne Zusatz |
| 2. BAL-Zellen bzw. PBMC | + Liposomen leer |
| 3. BAL-Zellen bzw. PBMC | + Liposomen VitA 5 µM final |
| 4. BAL-Zellen bzw. PBMC | + VitA pur 5 µM final |

### Messung von MMP-9 und TIMP-1 auf Proteinebene im ELISA

Für die Messung von MMP-9 und TIMP-1 in den Zellkulturüberständen werden kommerziell erhältliche ELISA Kits der Firma Amersham Pharmacia Biotech (Freiburg, Germany) verwendet. Im einzelnen sind dies:
Matrixmetalloproteinase-9 (MMP-9), human, ELISA System: code RPN 2614
Tissue inhibitor of metalloproteinases-1 (TIMP-1), human, ELISA System: code RPN 2611
Matrixmetalloproteinase-9 (MMP-9), human, activity system: code RPN 2630
Die Durchführung der ELISA's erfolgt jeweils genau nach Herstellerangaben.

### Bestimmung der mRNA-Expression von MMP-9 und TIMP-1 mittels semiquantitativer RT-PCR

Die verwendete Methode ist etabliert und bereits mehrfach publiziert. Die Beschreibung erfolgt deshalb nur stichpunktartig.
- Isolierung der Gesamt-RNA nach der RNAclean Methode
- umschreiben der RNA in cDNA mit oligo-dT
- Amplifikation der cDNA mit spezifischen Primern für MMP-9, TIMP-1 und α-Enolase als Kontrolle
- Auswertung über ein Agarosegel

### Herstellung von Liposomen mit all trans Retinsäure 5 × 10⁻⁴ M

### Reagenzien:

- Phoshatidylcholin: 1-Palmitoyl-2-oleyl-sn-glycero-3-phosphocholin = POPC
- Phosphatidylserin: 1,1-Dioleyl-sn-glycero-3phospho-L-serin-Mononatriumsalz = OOPS
- all trans-Retinsäure (Vitamin A): Sigma # R-2625 (Deisenhofen, Germany) 10 mM Stammlösung in Ethanol absolut
- (±)-α-Tocopherol (Vitamin E): Sigma # T-3251 (Deisenhofen) Stammlösung 20 mg/ml in Ethanol absolut

Bei allen verwendeten Reagenzien auf Endotoxin-Freiheit (LPS-Freiheit) achten, bzw. alle Glaswaren durch 3h Backen bei 180 °C LPS-frei bringen. Zur Herstellung von 1 ml Liposomen 17.5 mg POPC und 7.5 mg OOPS in ein steriles Einfrierröhrchen (NUNC) einwiegen. Durch Zugabe von 1 ml Chloroform (Merck) die Lipide lösen. Die gesamte Lösung in einen 250 ml Duranglas-Rundkolben überführen und 50 µl der Vitamin A Stammlösung dazupipettieren. Als Oxidationsschutz für die Lipide und das Vitamin A ferner 10 µl der Vitamin E Stammlösung zugeben. Unter sterilen Bedingungen, während leichtem Erwärmen (nicht über 42 °C) und unter Rotation des Kolbens das Chloroform verdampfen, bis sich ein weißlicher Lipidfilm an der Glaswand bildet. Durch Zugabe von 1 ml sterilem LPS- freien Phosphatpuffer (PBS) die Liposomen unter Schütteln des Kolbens rekonstituieren. Zur vollständigen Rekonstitution der Liposomen die Präparation für ca. 20 min bei Raumtemperatur (lichtgeschützt) ruhen lassen. Danach diese so entstandenen multilamellären Liposomen in ein steriles 1.5 ml Eppendorf Reaktionsgefäß überführen. Zur Kontrolle stets 'leere' Liposomen ohne Zugabe von Vitamin A herstellen. Zur Entfernung des nicht in die Liposomen verpackten Vitamin A die Liposomenpräparation für 5 min bei 14.000 x g in einer Eppendorf Tischzentrifuge abzentrifugieren. Den Überstand abpipettieren und das Volumen mit frischem PBS wieder auf 1 ml ergänzen. Diesen Waschschritt insgesamt 6 mal durchführen.

In anschließendem Extrusions-Prozeß die multilamellären Liposomen zu unilamellären Liposomen von 0.4 µm Durchmesser bringen. Dazu den Extruder gemäß den Angaben des Herstellers (Lipex Biomembranes, Vancouver, Canada) zusammenbauen. Für den Erhalt von 0.4 µm Liposomen zum Vorfilter (Drain Disc, Costar # 230 300) einen Filter der Porengröße 0.4 µm (Costar Nuclepore # 110 407) einsetzen. Das gesamte Liposomenvolumen mit einer Pasteurpipette oben in das Gerät einfüllen. Unter Druck (ca. 5-10 bar, nicht höher als 40 bar) die Liposomen extruieren und in einem Eppendorf Reaktionsgefäß auffangen. Diese Liposomen erneut oben in das Gerät einfüllen und Vorgang wiederholen (der Membranfilter kann bis zu 9 mal verwendet werden). Eine Liposomenpräparation muß diesen Zyklus mindestens dreimal (besser fünf mal) durchlaufen. Die fertigen unilamellären Liposomen unter der Sterilbank für die Verwendung in Zellkultur durch einen Millipore # SLGV 013 OS Filter sterilfiltrieren. Eine so hergestellte Liposomenpräparation ist für mindestens vier Wochen verwendbar.

### Charakterisierung der Liposomen

- unilamellär
- Duchmesser von 0.4 µm
- Überprüfung der Funktionalität mit Hilfe eines speziellen Färbeverfahrens und der Analyse im Durchflusszytometer möglich (kann bei Bedarf ausführlich beschrieben werden)

## Patentansprüche

1. Pharmazeutische Zusammensetzung in Form eines Aerosol-Inhalats, enthaltend als Wirkstoffe Vitamin A und/oder seine Derivate und/oder Ester in einer therapeutisch wirksamen Menge, **dadurch gekennzeichnet, daß** die Wirkstoffe in unilamellären Liposomen mit einer Größe von 0,1 µm bis 2 µm eingeschlossen sind und die Liposomen zumindest Phosphatidylcholin und ein Phospholipid, ausgewählt aus der Gruppe, bestehend aus Phosphatidylinositol, Phosphatidylserin, Phosphatidinsäure und Cardiolipin, in ihrer Lipid-Doppelschicht enthalten, und das Verhältnis des Phosphatidylcholins zum weiteren Phospholipid 65:35 bis 75:25 beträgt.

2. Pharmazeutische Zusammensetzung nach Anspruch 1,
**dadurch gekennzeichnet,**
**daß** das Vitamin A Retinol, Retinal und/oder Retinsäure und ihre Derivate und Ester und β-Carotin und deren Derivate als Vorstufe von Vitamin A ist.

3. Pharmazeutische Zusammensetzung nach einem oder mehreren der vorhergehenden Ansprüche,
**dadurch gekennzeichnet,**
**daß** die Liposomen einen Stabilisator enthalten.

4. Pharmazeutische Zusammensetzung nach Anspruch 3,
**dadurch gekennzeichnet,**
**daß** als Stabilisator ein Antioxidationsmittel eingesetzt wird.

5. Pharmazeutische Zusammensetzung nach Anspruch 3 oder 4,
**dadurch gekennzeichnet,**
**daß** als Stabilisator Vitamin E eingesetzt wird.

6. Pharmazeutische Zusammensetzung nach Anspruch 3, 4 oder 5,
**dadurch gekennzeichnet,**
**daß** der Stabilisator in einer Menge von 0.1 - 10%, bevorzugt 0.8%, bezogen auf 25 mg Lipide, eingesetzt wird.

7. Pharmazeutische Zusammensetzung nach einem oder mehreren der vorhergehenden Ansprüche,
**dadurch gekennzeichnet,**
**daß** neben Phosphatidylcholin Phosphatidylserin vorliegt.

8. Pharmazeutische Zusammensetzung nach einem oder mehreren der vorhergehenden Ansprüche,
**dadurch gekennzeichnet,**
**daß** die Liposomen 5.0 mg bis 0.0015 mg an Vitamin A und/oder seinen Derivaten und Estern, bezogen auf 25 mg Lipide, bevorzugt 0.05 mg - 0.30 mg Vitamin A pro 25 mg Lipide, enthalten.

9. Pharmazeutische Zusammensetzung nach einem oder mehreren der vorhergehenden Ansprüche,
**dadurch gekennzeichnet,**
**daß** sie weiterhin Träger- und/oder Hilfsstoffe enthält.

10. Pharmazeutische Zusammensetzung nach einem oder mehreren der vorhergehenden Ansprüche,
**dadurch gekennzeichnet,**
**daß** sie neben Vitamin A weitere Wirkstoffe enthält.

11. Verwendung von Vitamin A und/oder seinen Derivaten und/oder Estern als Wirkstoffe in einer therapeutisch wirksamen Menge, wobei die Wirkstoffe in unilamellären Liposomen mit einer Größe von 0,1 µm bis 2 µm eingeschlossen sind und die Liposomen zumindest Phosphatidylcholin und ein weiteres Phospholipid, ausgewählt aus der Gruppe, bestehend aus Phosphatidylinositol, Phosphatidylserin, Phosphatidinsäure und Cardiolipin, in ihrer Lipid-Doppelschicht enthalten, und das Verhältnis des Phosphatidylcholins zum weiteren Phospholipid 65:35 bis 75:25 beträgt, zur Herstellung einer pharmazeutischen Zusammensetzung in Form eines Aerosol-Inhalats zur Prophylaxe und Therapie von Erkrankungen des Tracheo-Bronchialtraktes.

12. Verwendung nach Anspruch 11 zur Therapie der COPD, der chronischen Brochitiden, des Emphysems und maligner sowie benigner Lungenerkrankungen

13. Verwendung einer pharmazeutischen Zubereitung in Form eines Aerosol-Inhalats, enthaltend zumindest Vitamin A und/oder seine Derivate und/oder seine Ester als Wirkstoff in einer therapeutisch wirksamen Menge zur Behandlung der COPD.

14. Verwendung nach Anspruch 13,
**dadurch gekennzeichnet,**
**daß** als Vitamin A Retinol, Retinal und/oder Retinsäure und ihre Derivate und Ester und/oder β-Carotin und seine Derivate als Vorstufe von Vitamin A eingesetzt werden.

## Claims

1. A pharmaceutical composition in the form of an aerosol inhalant containing as active agents vitamin A and/or the derivatives thereof and/or esters thereof in a therapeutically effective amount
**characterized in that**
said active agents are enclosed in unilamellar liposomes having a size of 0.1*µ*m to 2*µ*m, and that said liposomes comprise at least phosphatidylcholine and another phospholipid selected from the group consisting of phosphatidylinositol, phosphatidylserine, phosphatidic acid and cardiolipin, in their lipid bilayer, and that the ratio of said phosphatidylcholine to said another phospholipid is from 65:35 to 75:25.

2. A pharmaceutical composition according to claim 1
**characterized in that**
said vitamin A is retinol, retinal and/or retinoic acid and the derivatives and esters thereof, and ß-carotene and the derivatives thereof being the precursor of vitamin A.

3. A pharmaceutical composition according to one or more of the preceding claims
**characterized in that**
said liposomes contain a stabilizer.

4. A pharmaceutical composition according to claim 3
**characterized in that**
an antioxidant is used as said stabilizer.

5. A pharmaceutical composition according to claim 3 or 4
**characterized in that**
vitamin E is used as said stabilizer.

6. A pharmaceutical composition according to claim 3, 4 or 5
**characterized in that**
said stabilizer is used in an amount of 0.1 - 10%, preferably 0.8%, based on 25 mg lipids.

7. A pharmaceutical composition according to one or more of the preceding claims
**characterized in that**
phosphatidylserine is present in addition to phosphatidylcholine.

8. A pharmaceutical composition according to one or more of the preceding claims
**characterized in that**
said liposomes contain 5.0 mg to 0.0015 mg vitamin A and/or the derivatives and esters thereof, based on 25 mg lipids, preferably 0.05 mg - 0.30 mg vitamin A per 25 mg lipids.

9. A pharmaceutical composition according to one or more of the preceding claims
**characterized in that**
said composition further contains carriers and auxiliary agents.

10. A pharmaceutical composition according to one or more of the preceding claims
**characterized in that**
said composition contains other active agents besides vitamin A.

11. Use of vitamin A and/or the derivatives thereof and/or the esters thereof as active agent in a therapeutically effective amount wherein said active agents are enclosed in unilamellar liposomes having a size of 0.1µm to 2µm, and wherein said liposomes comprise at least phosphatidylcholine and another phospholipid selected from the group consisting of phosphatidylinositol, phosphatidylserine, phosphatidic acid and cardiolipin, in their lipid bilayer, and wherein the ratio of said phosphatidylcholine to said another phospholipid is from 65:35 to 75:25 for the preparation of a pharmaceutical composition in the form of an aerosol inhalant for the prophylaxis and the treatment of tracheo-bronchial tract disorders.

12. The use according to claim 11 for the therapy of COPD, chronic bronchitides, emphysema, and malignant as well as benign lung diseases.

13. The use of a pharmaceutical preparation in the form of an aerosol inhalant containing at least vitamin A and/or the derivatives thereof and/or the esters thereof as the active agent in a therapeutically effective amount for the treatment of COPD.

14. The use according to claim 13
**characterized in that**
retinol, retinal and/or retinoic acid and the derivatives and esters thereof, and/or ß-carotene and the derivatives thereof being the precursor of vitamin A are used as vitamin A.

## Revendications

1. Composition pharmaceutique sous la forme d'un aérosol à inhaler, contenant comme principes actifs de la vitamine A et/ou ses dérivés et/ou ses esters en une quantité efficace thérapeutiquement, **caractérisée en ce que** les principes actifs sont inclus dans des liposomes unilamellaires d'une dimension de 0,1 µm à 2 µm et les liposomes contiennent au moins de la phosphatidylcholine et un phospholipide choisi dans le groupe constitué du phosphatidylinositol, de la phosphatidylsérine, de l'acide phosphatidinique et de la cardiolipine dans leurs doubles couches lipidiques et le rapport de la phosphatidylcholine à l'autre phospholipide est compris entre 65:35 et 75:25.

2. Composition pharmaceutique suivant la revendication 1,
**caractérisée**
**en ce que** la vitamine A est, en tant que précurseur de la vitamine A, du rétinol, du rétinal et/ou de l'acide rétinique et ses dérivés et esters et de la β-carotine et ses dérivés.

3. Composition pharmaceutique suivant l'une ou plusieurs des revendications précédentes,
**caractérisée**
**en ce que** les liposomes contiennent un agent de stabilisation.

4. Composition pharmaceutique suivant la revendication 3,
**caractérisée**
**en ce que** l'on utilise un agent antioxydant comme agent de stabilisation.

5. Composition pharmaceutique suivant la revendication 3 ou 4,
**caractérisée**
**en ce que** l'on utilise de la vitamine E comme agent de stabilisation.

6. Composition pharmaceutique suivant la revendication 3, 4 ou 5,
**caractérisée**
**en ce que** l'on utilise l'agent de stabilisation en une quantité de 0,1 à 10 %, de préférence de 0,8 % rapportée à 25 mg de lipides.

7. Composition pharmaceutique suivant l'une ou plusieurs des revendications précédentes,
**caractérisée**
**en ce qu'**il y a, outre de la phosphatidylcholine, de la phosphatidylsérine.

8. Composition pharmaceutique suivant l'une ou plusieurs des revendications précédentes,
**caractérisée**
**en ce que** les liposomes contiennent de 5,0 mg à 0,0015 mg de vitamine A et/ou de ses dérivés et esters, rapporté à 25 mg de lipides, de préférence de 0,05 mg à 0,30 mg de vitamine A par 25 mg de lipides.

9. Composition pharmaceutique suivant l'une ou plusieurs des revendications précédentes,
**caractérisée**
**en ce qu'**elle contient, en outre, des véhicules et/ou des adjuvants.

10. Composition pharmaceutique suivant l'une ou plusieurs des revendications précédentes,
**caractérisée**
**en ce qu'**elle contient d'autres principes actifs en plus de la vitamine A.

11. Utilisation de la vitamine A et/ou de ses dérivés et/ou de ses esters comme principes actifs en une quantité efficace thérapeutiquement, les principes actifs étant inclus dans des liposomes unilamellaires d'une dimension de 0,1 µm à 2 µm et les liposomes contenant au moins de la phosphatidylcholine et un phospholipide choisi dans le groupe constitué du phosphatidylinositol, de la phosphatidylsérine, de l'acide phosphatidinique et de la cardiolipine dans leurs doubles couches lipidiques et le rapport de la phosphatidylcholine à l'autre phospholipide est compris entre 65:35 et 75:25 pour la préparation d'une composition pharmaceutique sous la forme d'un aérosol à inhaler pour la prophylaxie et la thérapie de maladies du tractus trachéobronchique.

12. Utilisation suivant la revendication 11 pour la thérapie du syndrome respiratoire obstructif chronique, des bronchites chroniques, de l'emphysème et des maladies pulmonaires malignes ainsi que bénignes.

13. Utilisation d'une préparation pharmaceutique sous la forme d'un aérosol à inhaler, comprenant au moins de la vitamine A et/ou ses dérivés et/ou ses esters comme principe actif en une quantité efficace thérapeutiquement pour le traitement du syndrome respiratoire obstructif chronique.

14. Utilisation suivant la revendication 13,
**caractérisée**
**en ce que** l'on utilise comme vitamine A du rétinol, du rétinol et/ou de l'acide rétinique et ses dérivés et ses esters et/ou de la β-carotine et ses dérivés comme précurseur de vitamine A.
